(19) Europäisches Patentamt
European Patent Office
Office européen des brevets

(11) Veröffentlichungsnummer: **0 075 093**
**B1**

(12) # EUROPÄISCHE PATENTSCHRIFT

(45) Veröffentlichungstag der Patentschrift: **05.12.84**

(51) Int. Cl.³: **C 07 D 207/16**

(21) Anmeldenummer: **82107088.5**

(22) Anmeldetag: **05.08.82**

(54) **Verfahren zur Herstellung von L-Prolin.**

(30) Priorität: **19.09.81 DE 3137377**

(43) Veröffentlichungstag der Anmeldung: **30.03.83 Patentblatt 83/13**

(45) Bekanntmachung des Hinweises auf die Patenterteilung: **05.12.84 Patentblatt 84/49**

(84) Benannte Vertragsstaaten: **AT BE CH DE FR GB IT LI NL**

(56) Entgegenhaltungen:
**SYNTHESIS, 1974, Georg Thieme Verlag, Stuttgart, Academic Press, New York, London, H.J. MONTEIRO "A convenient synthesis of L-Proline"**
**CHEMICAL ABSTRACTS, Band 49, (1955), Columbus, Ohio, USA, Z. PRAVDA et al. "Amino acids and peptides. XIII. Synthesis of L-proline from L-glutamic acid", Zusammenfassung No. 14 640 d**
**CHEMISTRY & INDUSTRY, weekly publication of the Society of Chemical Industry, 1966, London, R. BUYLE "Synthesis of L-Proline and N-Methyl-L-Proline from L-Glutamic Acid"**
**BERICHTE DER DEUTSCHEN CHEMISCHEN GESELLSCHAFT, 44. Jahrgang, Band II, 1911, Berlin, EMIL FISCHER & REGINALD BOEHNER "Verwandlung der Glutaminsäure bzw. Pyrrolidon-carbonsäure in Prolin"**

(73) Patentinhaber: **Degussa Aktiengesellschaft, Weissfrauenstrasse 9, D-6000 Frankfurt am Main 1 (DE)**

(72) Erfinder: **Drauz, Karlheinz, Dr., Flurstrasse 5, D-6463 Freigericht (DE)**
Erfinder: **Effenberger, Franz, Prof.Dr., Schatzweg 5, D-7000 Stuttgart 70 (DE)**
Erfinder: **Kleemann, Axel. Dr., Greifenhagenstrasse, D-6450 Hanau 9 (DE)**
Erfinder: **Martens, Jürgen, Dr., Hochstrasse 5, D-8755 Alzenau (DE)**
Erfinder: **Scherberich, Paul, Dr., Königsberger Strasse 8, D-7750 Konstanz (DE)**

**EP 0 075 093 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Gegenstand der Erfindung ist ein neues Verfahren zur Herstellung von L-Prolin aus einem L-Pyroglutaminsäureester.

Es sind bereits verschiedene Verfahren zur Herstellung von L-Prolin aus dem Methyl- oder Ethylester der L-Pyroglutaminsäure bekannt. Die bekannten Verfahren ergeben jedoch L-Prolin nur in geringen Ausbeuten.

Es ist ferner auch bereits bekannt, L-Prolin durch Umsetzung von freier L-Pyroglutaminsäure mit Triethyloxoniumtetrafluoroborat und anschliessende Reduktion des erhaltenen Iminoethers mit Natriumborhydrid herzustellen („Synthesis", 1974, S. 137). Als Ausbeute werden 75% angegeben. Abgesehen davon, dass diese Ausbeute von anderen Autoren nicht reproduziert werden konnte (vgl. z.B. D. Enders *et al.* in „Chem. Ber.", *112*, S. 3714 [1979], Fussnote 19), ist das in grossem Überschuss benötigte Triethyloxoniumtetrafluoroborat nur sehr schwierig zu handhaben, so dass eine Übertragung dieses Verfahrens in den technischen Massstab nicht möglich erscheint.

Das erfindungsgemässe Verfahren ist nun dadurch gekennzeichnet, dass man

a) einen L-Pyroglutaminsäureester der allgemeinen Formel:

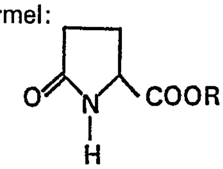

(I)

in der R einen Methyl- oder Ethylrest bedeutet, in einem inerten Lösungsmittel mit mindestens 2 mol pro Mol eingesetzter Verbindung der allgemeinen Formel I an Phosgen zur Reaktion bringt und anschliessend das Lösungsmittel und überschüssiges Phosgen durch Destillation abtrennt,

b) den in der Reaktionsstufe a erhaltenen Rückstand in einem inerten Lösungsmittel mit mindestens 1 mol pro Mol ursprünglich eingesetzter Verbindung der allgemeinen Formel I eines Säureacceptors 3 bis 10 h lang auf eine Temperatur zwischen 60 und 100°C erhitzt,

c) das in der Reaktionsstufe b erhaltene rohe Reaktionsgemisch oder die daraus durch Destillation isolierte und in einem inerten Lösungsmittel gelöste Verbindung der allgemeinen Formel:

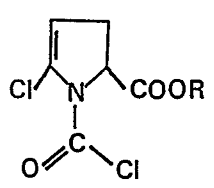

(III)

in der R wieder einen Methyl- oder Ethylrest bedeutet, in Gegenwart eines Hydrierkatalysators und der mindestens stöchiometrischen Menge eines Säureacceptors bei einer Temperatur zwischen 0 und 100°C und einem Wasserstoffdruck zwischen 1 und 300 bar hydriert und anschliessend den Hydrierkatalysator durch Filtration abtrennt,

d) das in der Reaktionsstufe c erhaltene rohe Reaktionsgemisch oder die daraus durch Destillation isoliert Verbindung der allgemeinen Formel:

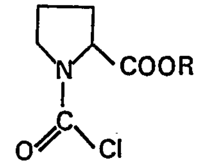

(IV)

in der R wieder einen Methyl- oder Ethylrest bedeutet, mit einer wässerigen Mineralsäure oder einem Gemisch aus einer wässerigen Mineralsäure und Ameisen- oder Essigsäure hydrolysiert, und

e) aus dem in der Reaktionsstufe d erhaltenen Hydrolysegemisch das enthaltene L-Prolin in an sich bekannter Weise isoliert.

Durch das erfindungsgemässe Verfahren kann der Methyl- oder Ethylester der L-Pyroglutaminsäure (Pyrrolidon-(2)-carbonsäure-(5)) unter Erhalt der Konfiguration am asymmetrischen Kohlenstoffatom leicht und in hoher Ausbeute in L-Prolin umgewandelt werden. Da sich die Methyl- und Ethylester der L-Pyroglutaminsäure ihrerseits nach bekannten Verfahren ebenfalls leicht und in hoher Ausbeute aus L-Glutaminsäure herstellen lassen, eröffnet das erfindungsgemässe Verfahren insgesamt einen neuen, vorteilhaften und kostengünstigen Weg zur Herstellung von L-Prolin aus L-Glutaminsäure.

Bei der Durchführung des erfindungsgemässen Verfahrens wird in einer ersten Verfahrensstufe der als Ausgangsmaterial dienende L-Pyroglutaminsäureester der allgemeinen Formel I in einem inerten Lösungsmittel mit mindestens der 2fachen, vorzugsweise der 2,01fachen bis 12fachen, insbesondere der 2,05fachen bis 3fachen, molaren Menge an Phosgen zur Reaktion gebracht. Die Reaktion erfolgt zweckmässigerweise bei einer Temperatur zwischen −30 und +50°C. Geeignete inerte Lösungsmittel sind beispielsweise Halogenkohlenwasserstoffe, wie Dichlormethan, Chloroform, Tetrachlorkohlenstoff oder 1,2-Dichlorethan; Ether, wie Di-n-propylether, Diisopropylether, Methyl-tert.-butylether, Tetrahydrofuran oder Dioxan; Carbonsäureester, wie Essigsäureethylester, Essigsäurepropylester, Essigsäurebutylester oder Propionsäureethylester; oder aromatische Kohlenwasserstoffe, wie Benzol oder Toluol. Bevorzugt werden Dichlormethan, Chloroform, 1,2-Dichlorethan oder die niederen Alkylester der Essigsäure verwendet.

Die erste Reaktionsstufe kann beispielsweise so durchgeführt werden, dass man zu der vorgelegten Lösung des Pyroglutaminsäureesters flüssiges (= kondensiertes) Phosgen zugibt. Diese Verfahrensweise wird vorteilhaft in dem Temperaturbereich zwischen −30 und +5°C vorgenommen. Das Phosgen kann entweder langsam zudosiert oder portionsweise oder auf einmal zugegeben werden. Nach beendeter Zugabe des Phosgens wird zweckmässigerweise noch etwa 1 h lang bei einer Temperatur im genannten Bereich gerührt. Anschliessend lässt man das Reaktionsgemisch vorteilhafterweise noch weitere 3 bis 12 h lang bei Raumtemperatur stehen.

Bei dieser Verfahrensweise entsteht aus dem Pyroglutaminsäureester der entsprechende 1-Chlorcarbonyl-5,5-dichlorprolinester der allgemeinen Formel:

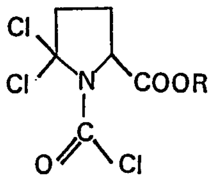

(II)

in der R einen Methyl- oder Ethylrest bedeutet, der durch vorsichtiges Abdestillieren des Lösungsmittels bei niedriger Temperatur, also gegebenenfalls unter vermindertem Druck, isoliert werden kann.

Die erste Reaktionsstufe kann aber beispielsweise auch so durchgeführt werden, dass man bei einer Temperatur zwischen 5 und 50° C einen kräftigen Strom von gasförmigen Phosgen in die Lösung des Pyroglutaminsäureesters einleitet. Wenn die erforderliche Menge an Phosgen zugeführt ist, wird zweckmässigerweise noch 0,5 bis 5 h lang bei der gewählten Reaktionstemperatur nachgerührt. Auch bei dieser Verfahrensweise entsteht zunächst der entsprechende 1-Chlorcarbonyl-5,5-dichlorprolinester der allgemeinen Formel II. Infolge der höheren Temperatur kann aber bereits spontan Chlorwasserstoffabspaltung eintreten, so dass ein Teil des 1-Chlorcarbonyl-5,5-dichlorprolinesters in den entsprechenden 2-Chlor-1-chlorcarbonylpyrrolin-(2)-carbonsäureester-(5) der allgemeinen Formel III übergeht.

Am Ende der ersten Reaktionsstufe wird das Lösungsmittel und überschüssiges Phosgen durch Destillation abgetrennt. Der Destillationsrückstand, unabhängig davon, ob er nun die reine Verbindung der allgemeinen Formel II oder ein Gemisch von Verbindungen der allgemeinen Formeln II und III enthält, wird nun in einer zweiten Reaktionsstufe in einem inerten Lösungsmittel mit mindestens 1 mol pro Mol ursprünglich eingesetzter Verbindung der allgemeinen Formel I eines Säureacceptors 3 bis 10 h lang auf eine Temperatur zwischen 60 und 100° C erhitzt, um die Abspaltung des Chlorwasserstoffes aus der Verbindung der allgemeinen Formel II zu vervollständigen. Geeignete inerte Lösungsmittel sind beispielsweise das bevorzugt verwendete 1,4-Dioxan, aber auch andere Ether, wie Di-n-propylether, Diisopropylether, Methyl-tert.-butylether oder Tetrahydrofuran; Carbonsäureester, wie Essigsäureethylester, Essigsäurepropylester, Essigsäurebutylester oder Propionsäureethylester; oder aromatische Kohlenwasserstoffe, wie Benzol oder Toluol. Geeignete Säureacceptoren sind insbesondere tertiäre Amine, wie Triethylamin, Tri-n-propylamin oder das besonders bevorzugte Tri-n-butylamin. Unter Umständen kann es vorteilhaft sein, die Chlorwasserstoffabspaltung in einer Inertgasatmosphäre, z.B. unter Stickstoff oder Argon, durchzuführen. Aus dem rohen Reaktionsgemisch der zweiten Reaktionsstufe können nun die reinen 2-Chlor-1-chlorcarbonylpyrrolin-(2)-carbonsäureester-(5) der allgemeinen Formel III durch Destillation, zweckmässigerweise unter vermindertem Druck, isoliert werden.

Die reinen Verbindungen der allgemeinen Formel III oder, was besonders vorteilhaft ist, das rohe Reaktionsgemisch der zweiten Reaktionsstufe werden nun in einer dritten Reaktionsstufe einer katalytischen Hydrierung unterworfen. Werden die reinen Verbindungen der allgemeinen Formel III eingesetzt, so müssen sie erneut in einem inerten Lösungsmittel gelöst werden. Geeignete inerte Lösungsmittel sind die bereits genannten in der zweiten Reaktionsstufe verwendeten. Als Hydrierkatalysatoren werden im allgemeinen die Metalle der Nebengruppe VIII des Periodensystems oder geeignete Verbindungen dieser Metalle bevorzugt. Die Metalle oder Metallverbindungen können als solche, aber auch in an sich bekannter Weise auf geeignete Trägermaterialien aufgebracht, in Form von Trägerkatalysatoren verwendet werden. Besonders bevorzugte Katalysatoren sind Palladium und/oder dessen Verbindungen, wie feinverteiltes Palladiummetall, insbesondere Palladiummohr, -chlorid, -bromid, -jodid, -nitrat, -oxid oder -oxidhydrat; oder Palladiumkomplexsalze, wie Tetra- oder Hexachloropalladate. Für Trägerkatalysatoren geeignete Trägermaterialien sind beispielsweise Aktivkohle, Kieselgele, Aluminiumoxid, Zeolithe, Bariumsulfat oder Calciumcarbonat. Die Hydrierkatalysatoren werden zweckmässigerweise in einer Menge zwischen 0,01 und 50, vorzugsweise zwischen 0,1 und 10 Gew.-%, berechnet als aktives Metall und bezogen auf das Gewicht der eingesetzten Verbindung der allgemeinen Formel III eingesetzt. Sofern das rohe Reaktionsgemisch der zweiten Reaktionsstufe direkt weiterverarbeitet wird, bezieht man die Katalysatormenge zweckmässig auf das Gewicht der aus der ursprünglich eingesetzten Menge der Verbindung der allgemeinen Formel I theoretisch zu erwartenden Menge an Verbindung der allgemeinen Formel III.

Die Hydrierungsreaktion erfordert ferner die Gegenwart einer der eingesetzten Menge an Verbindung der allgemeinen Formel III mindestens stöchiometrischen Menge eines Säureacceptors. Sofern das rohe Reaktionsgemisch der zweiten Reaktionsstufe direkt der Hydrierung unterworfen wird, legt man zweckmässigerweise wieder den theoretisch zu erwartenden Gehalt an Verbindung der allgemeinen Formel III zugrunde. In diesem Falle ist es darüber hinaus besonders vorteilhaft, wenn man schon vor der Druchführung der zweiten Reaktionsstufe eine auch für die dritte Reaktionsstufe ausreichende Menge an Säureacceptor zusetzt. Besonders vorteilhaft ist dann der Zusatz von 2,01 bis 10 mol Säureacceptor pro Mol ursprünglich eingesetzter Verbindung der allgemeinen Formel I. Geeignete Säureacceptoren sind die bereits genannten in der zweiten Reaktionsstufe verwendeten.

Die Hydrierungsreaktion wird in der für Hydrierungen üblichen Weise bei einer Temperatur zwischen 0 und 100, vorzugsweise zwischen 25 und 80° C, und einem Wasserstoffdruck zwischen 1 und 300, vorzugsweise zwischen 75 und 200 bar,

vorgenommen. Nach beendeter Hydrierung wird der Hydrierkatalysator durch Filtration aus dem Reaktionsgemisch abgetrennt.

Bei der Hydrierungsreaktion entsteht aus dem eingesetzten 2-Chlor-1-chlorcarbonylpyrrolin-(2)-carbonsäureester-(5) der allgemeinen Formel III der entsprechende N-Chlorcarbonylprolinester der allgemeinen Formel IV. Er kann durch Destillation, zweckmässigerweise unter vermindertem Druck, aus dem rohen Reaktionsgemisch isoliert werden.

Die Verbindung der allgemeinen Formel IV sind neu und stellen unter anderem wertvolle Zwischenprodukte für die Herstellung an sich bekannter Pestizide dar.

Die reinen Verbindungen der allgemeinen Formel IV oder, was wieder besonders vorteilhaft ist, das rohe Reaktionsgemisch der dritten Reaktionsstufe werden nun in einer vierten Reaktionsstufe mit einer wässerigen Mineralsäure oder einem Gemisch aus einer wässerigen Mineralsäure und Ameisen- oder Essigsäure hydrolysiert. Bevorzugt wird die Verwendung von wässeriger Salzsäure oder von Gemischen aus wässeriger Salzsäure und Ameisen- oder Essigsäure. Wird das rohe Reaktionsgemisch aus der dritten Reaktionsstufe direkt der Hydrolyse unterworfen, so ist es zweckmässig, wenn dessen Gehalt an überschüssigem Säureacceptor möglichst gering ist. Die Hydrolyse erfolgt in der für Hydrolysen üblichen Weise mit einem Überschuss an Hydrolysemittel bei einer Temperatur zwischen 50°C und der Rückflusstemperatur des Reaktionsgemisches. Sie erfordert im allgemeinen eine Reaktionszeit zwischen 1 und 12 h. Unter Umständen ist es vorteilhaft, das Hydrolysegemisch anschliessend noch einige Zeit, beispielsweise 5 h, bei Raumtemperatur stehenzulassen.

Bei der Hydrolyse wird aus der eingesetzten Verbindung der allgemeinen Formel IV gleichzeitig die Chlorcarbonylgruppe abgespalten und die Estergruppe verseift, so dass L-Prolin entsteht, das im rohen Hydrolysegemisch als Additionssalz der eingesetzten Mineralsäure vorliegt und aus diesem in an sich bekannter Weise, z.B. mittels eines Ionenaustauscherharzes, isoliert werden kann.

In den nachfolgenden Beispielen wird das erfindungsgemässe Verfahren näher erläutert. Prozentangaben bedeuten, sofern nichts anderes angegeben ist, Gewichtsprozente.

*Beispiel 1:*

35,8 g L-Pyroglutaminsäuremethylester werden in 300 ml Methylenchlorid gelöst und auf −10°C abgekühlt. In einen kalibrierten, gekühlten Tropftrichter werden 74,8 g Phosgen einkondensiert. Unter Rühren und Feuchtigkeitsausschluss gibt man das flüssige Phosgen auf einmal zu der Reaktionslösung, rührt 1 h bei −10°C nach und lässt anschliessend 12 h bei Raumtemperatur stehen.

Nach Abdampfen des Lösungsmittels und des überschüssigen Phosgens wird der Rückstand in 300 ml Dioxan gelöst und mit 110 g Tri-n-butylamin versetzt. Diese Reaktionsmischung wird in einer Stickstoffatmosphäre unter Rühren 3 h bei 75°C gehalten.

Die klare Lösung wird anschliessend in einem Hastelloy-Rührautoklaven unter Zusatz von 32 g Palladium auf Aktivkohle (10%ig) bei 50°C und einem Wasserstoffdruck von 150 bar bis zur vollständigen Wasserstoffaufnahme hydriert. Nach Abfiltrieren des Katalysators wird die rohe Reaktionsmischung unter kräftigem Rühren zu 600 ml einer 75°C warmen, viertelskonzentrierten Salzsäure gegeben. Nach Beendigung der Gasentwicklung wird noch weitere 3 h bei 75°C gerührt. Man stellt diese Lösung mit Natriumhydroxid alkalisch, trennt das Tri-n-butylamin ab, und dampft die wässerige Phase nach einer Aktivkohleklärung ein. Der Rückstand wird in verdünnter Salzsäure aufgenommen, nochmals mit Aktivkohle geklärt, eingedampft und der Rückstand mit Hilfe eines basischen Ionenaustauschers in Prolin überführt. Man erhält 24,3 g (84,5%) L-Prolin mit einem Schmelzpunkt von 227-231°C (Zers.), $\alpha_D^{20} = 83,6°$ (c = 1, $H_2O$).

*Beispiel 2:*

Zu 22,5 g L-Pyroglutaminsäureethylester, gelöst in 150 ml Methylenchlorid, wird bei 45°C 3 h lang ein kräftiger Phosgenstrom eingeleitet. Danach bleibt das Reaktionsgemisch weitere 3 h bei Raumtemperatur stehen.

Nach Abdampfen des Lösungsmittels wird der Rückstand in 300 ml Tri-n-butylamin 3 h auf 70°C erwärmt und diese Reaktionsmischung unter Zusatz von 17 g Palladium auf Aktivkohle (10%ig) bei 50°C und einem Wasserstoffdruck von 180 bar im Autoklaven bis zur vollständigen Umsetzung hydriert.

Nach Hydrolyse und Aufarbeitung gemäss Beispiel 1 erhält man 12,8 g (78%ig) L-Prolin mit einem Schmelzpunkt von 228-230°C (Zers.).

*Beispiel 3:*

30,0 g L-Pyroglutaminsäuremethylester werden in 210 ml Methylenchlorid gelöst und mit 60,0 g Phosgen gemäss Beispiel 1 umgesetzt.

Nach Abdampfen des Lösungsmittels und des überschüssigen Phosgens erhält man einen Rückstand von 54,0 g. Eine kleine Probe dieses Rückstands wird zur Charakterisierung aus Diisopropylether umkristallisiert und erweist sich als L-1-Chlorcarbonyl-5,5-dichlorprolinmethylester mit einem Schmelzpunkt von 76-78°C.

Diese neue Verbindung liefert eine korrekte Elementaranalyse:

$C_7H_8Cl_3NO_3$ (260,5)

Berechnet: C 32,37 H 3,10 Cl 40,83 N 5,38%

Gefunden: C 32,48 H 3,17 Cl 40,56 N 5,55%

Ebenso sind die NMR- und IR-Spektren mit dieser Struktur in Übereinstimmung.

*Beispiel 4:*

52,1 g des im Beispiel 3 erhaltenen Rückstands werden mit 21,5 g Triethylamin in 500 ml Dioxan 5 h bei 80°C gerührt. Die Reaktion wird unter einer Stickstoffatmosphäre durchgeführt. Nach Reaktionsende wird das feste Triethylaminhydrochlorid

abgesaugt, das Dioxan entfernt und der Rückstand im Vakuum fraktioniert.

Bei 0,013 mbar und 110-118°C gehen 41,2 g (92%) L-2-Chlor-1-chlorcarbonylpyrrolin-(2)-carbonsäuremethylester-(5) über.

Diese neue Verbindung liefert korrekte IR- und NMR-Spektren.

*Analyse für* $C_7H_7Cl_2NO_3$ (224,04)

Berechnet: C 37,53 H 3,15 Cl 31,65 N 6,25%
Gefunden: C 37,75 H 3,01 Cl 31,78 N 6,34%

*Beispiel 5:*

35,8 g des im Beispiel 4 isolierten L-2-Chlor-1-chlorcarbonylpyrrolin-(2)-carbonsäuremethylesters-(5) werden in 200 ml Dioxan gelöst und nach Zugabe von 30 g Tri-n-butylamin und 18,5 Palladium auf Aktivkohle (10%ig) bei 50°C und 125 bar Wasserstoffdruck bis zur vollständigen Wasserstoffaufnahme hydriert. Nach Abtrennung des Katalysators, der Ammoniumsalze, des Lösungsmittels und des überschüssigen Tri-n-butylamins wird der Rückstand im Vakuum fraktioniert.

Man erhält 24,2 g (79%) L-N-Chlorcarbonylprolinmethylester mit einem Siedepunkt von 90-95°C bei 0,013 mbar.

Von dieser neuen Verbindung wurden korrekte IR- und NMR-Spektren erhalten.

*Analyse für* $C_7H_{10}Cl\,NO_3$ (191,62)

Berechnet: C 43,88 H 5,26 Cl 18,50 N 7,31%
Gefunden: C 44,16 H 5,46 Cl 17,93 N 7,38%

*Beispiel 6:*

19,2 g des im Beispiel 5 isolierten L-N-Chlorcarbonylprolinmethylesters werden zu 200 ml einer auf 75°C vorgewärmten, viertelskonzentrierten Salzsäure unter starkem Rühren zugetropft. Nach Ende der Gasentwicklung rührt man 1 h bei 75°C nach und lässt noch 5 h bei Raumtemperatur stehen. Die salzsaure Lösung wird eingedampft, und der Rückstand mittels eines schwach basischen Ionenaustauschers dehydrohalogeniert. Nach Eindampfen des Eluats erhält man 11,2 g (97,4%) L-Prolin mit einem Schmelzpunkt von 222-229°C (Zers.), $\alpha_D^{20} = 83{,}6°$ (c = 1, $H_2O$).

*Beispiel 7:*

50 g L-Pyroglutaminsäureethylester werden in 350 ml Methylenchlorid gelöst und bei −10°C gemäss Beispiel 1 mit 95 g Phosgen umgesetzt.

Das Lösungsmittel und das überschüssige Phosgen werden unter vermindertem Druck abgedampft, wobei ein Ansteigen der Temperatur über 30°C vermieden wird.

Eine kleine Probe des erhaltenen Rückstands erwies sich nach den NMR- und IR-Spektren und nach den elementaranalytischen Daten als reiner L-1-Chlorcarbonyl-5,5-dichlorprolinethylester.

*Analyse für* $C_8H_{10}Cl_3NO_3$ (274,53)

Berechnet: C 35,00 H 3,67 Cl 38,74 N 5,10%
Gefunden: C 35,24 H 3,60 Cl 38,72 N 5,07%

*Beispiel 8:*

87,2 g des im Beispiel 7 erhaltenen Rückstands werden gemäss Beispiel 4 mit 40 g Triethylamin in 500 ml Dioxan umgesetzt. Nach der Aufarbeitung ergibt die Destillation 61,3 g (81%) L-2-Chlor-1-chlorcarbonylpyrrolin-(2)-carbonsäureethylester-(5) mit einem Siedepunkt von 120-124°C bei einem Druck von 0,06 mbar.

Diese neue Verbindung liefert korrekte IR- und NMR-Spektren und ergibt übereinstimmende elementaranalytische Daten.

*Analyse für* $C_8H_9Cl_2NO_3$ (238,07)

Berechnet: C 40,36 H 3,31 Cl 29,78 N 5,88%
Gefunden: C 40,56 H 2,89 Cl 29,50 N 5,91%

*Beispiel 9:*

45,0 g L-Pyroglutaminsäureethylester werden in 300 ml Methylenchlorid gelöst, und bei 45°C wird 3 h lang ein kräftiger Phosgenstrom eingeleitet.

Nach Reaktionsende wird weitere 3 h bei 45°C gerührt. Nach Abdampfen des Lösungsmittels erhält man als Rückstand ein farbloses Öl, dessen Zusammensetzung NMR-spektroskopisch zu etwa 70% L-1-Chlorcarbonyl-5,5-dichlorprolinethylester und 30% L-2-Chlor-1-chlorcarbonylpyrrolin-(2)-carbonsäureethylester-(5) bestimmt wurde.

Das Öl wird in 400 ml Dioxan gelöst und nach Zugabe von 32 g Triethylamin unter Rühren 5 h bei 75°C unter einer Stickstoffatmosphäre gehalten. Nach Reaktionsende wird das Triethylaminhydrochlorid abgesaugt, das Filtrat eingedampft, und der Rückstand im Vakuum fraktioniert.

Bei einem Druck von 0,06 mbar gehen 53,8 g (79%) L-2-Chlor-1-chlorcarbonylpyrrolin-(2)-carbonsäureethylester-(5) bei 120-124°C über.

*Beispiel 10:*

47,6 g des im Beispiel 8 bzw. 9 isolierten L-2-Chlor-1-chlorcarbonylpyrrolin-(2)-carbonsäureethylesters-(5) werden gemäss Beispiel 5 bei einem Wasserstoffdruck von 180 bar hydriert. Nach Destillation erhält man 30,0 g (73%) L-N-Chlorcarbonylprolinethylester mit einem Siedepunkt von 90°C bei 0,001 mbar. Diese neue Verbindung wurde durch spektroskopische und elementaranalytische Daten charakterisiert.

*Analyse für* $C_8H_{12}Cl\,NO_3$ (205,64)

Berechnet: C 47,73 H 5,88 Cl 17,24 N 6,81%
Gefunden: C 46,80 H 6,03 Cl 17,20 N 7,21%

*Beispiel 11:*

20,6 g des im Beispiel 10 isolierten L-N-Chlorcarbonylprolinethylesters werden gemäss Beispiel 6 mit wässeriger Salzsäure umgesetzt. Nach Aufarbeitung erhält man 11,3 g (98,2%) L-Prolin mit einem Schmelzpunkt von 224-230°C (Zers.), $\alpha_D^{20} = -83{,}7°$ (c = 1, $H_2O$).

**Patentansprüche**

1. Verfahren zur Herstellung von L-Prolin aus

einem L-Pyroglutaminsäureester, dadurch gekennzeichnet, dass man

a) einen L-Pyroglutaminsäureester der allgemeinen Formel:

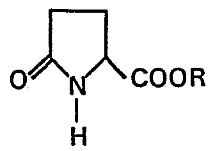

(I)

in der R einen Methyl- oder Ethylrest bedeutet, in einem inerten Lösungsmittel mit mindestens 2 mol pro Mol eingesetzter Verbindung der allgemeinen Formel I än Phosgen zur Reaktion bringt und anschliessend das Lösungsmittel und überschüssiges Phosgen durch Destillation abtrennt,

b) den in der Reaktionsstufe (a) erhaltenen Rückstand in einem inerten Lösungsmittel mit mindestens 1 mol pro Mol ursprünglich eingesetzter Verbindung der allgemeinen Formel (I) eines Säureacceptors 3 bis 10 h lang auf eine Temperatur zwischen 60 und 100°C erhitzt,

c) das in der Reaktionsstufe (b) erhaltene rohe Reaktionsgemisch oder die daraus durch Destillation isolierte und in einem inerten Lösungsmittel gelöste Verbindung der allgemeinen Formel:

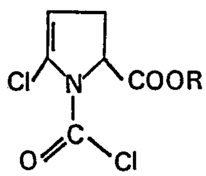

(III)

in der R wieder einen Methyl- oder Ethylrest bedeutet, in Gegenwart eines Hydrierkatalysators und der mindestens stöchiometrischen Menge eines Säureacceptors bei einer Temperatur zwischen 0 und 100°C und einem Wasserstoffdruck zwischen 1 und 300 bar hydriert und anschliessend den Hydrierkatalysator durch Filtration abtrennt,

d) das in der Reaktionsstufe (c) erhaltene rohe Reaktionsgemisch oder die daraus durch Destillation isolierte Verbindung der allgemeinen Formel:

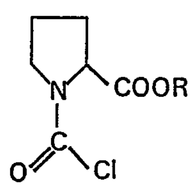

(IV)

in der R wieder einen Methyl- oder Ethylrest bedeutet, mit einer wässerigen Mineralsäure oder einem Gemisch aus einer wässerigen Mineralsäure und Ameisen- oder Essigsäure hydrolysiert, und

e) aus dem in der Reaktionsstufe (d) erhaltenen Hydrolysegemisch das enthaltene L-Prolin in an sich bekannter Weise isoliert.

2. Verfahren nach Anspruch 1, dadurch gekennzeichnet, dass man die Reaktionsstufe (a) bei einer Temperatur zwischen −30 und +5°C durchführt und das Phosgen in kondensierter Form zugibt.

3. Verfahren nach Anspruch 1, dadurch gekennzeichnet, dass man die Reaktionsstufe (a) bei einer Temperatur zwischen 5 und 50°C durchführt und gasförmiges Phosgen einleitet.

4. Verfahren nach einem der Ansprüche 1 bis 3, dadurch gekennzeichnet, dass man das Phosgen in einer Menge zwischen 2,0 und 12 mol pro Mol eingesetzter Verbindung der allgemeinen Formel (I) einsetzt.

5. Verfahren nach einem der Ansprüche 1 bis 4, dadurch gekennzeichnet, dass man in der Reaktionsstufe (b) als Säureacceptor ein tertiäres Amin einsetzt.

6. Verfahren nach einem der Ansprüche 1 bis 5, dadurch gekennzeichnet, dass man in der Reaktionsstufe (c) als Hydrierkatalysator ein Metall der Nebengruppe VIII des Periodensystems oder eine Verbindung eines solchen Metalls einsetzt.

7. Verfahren nach einem der Ansprüche 1 bis 6, dadurch gekennzeichnet, dass man in der Reaktionsstufe (c) als Säureacceptor ein tertiäres Amin einsetzt.

8. Verfahren nach einem der Ansprüche 1 bis 7, dadurch gekennzeichnet, dass man in der Reaktionsstufe (d) als Hydrolysemittel wässerige Salzsäure oder ein Gemisch von wässeriger Salzsäure und Ameisen- oder Essigsäure einsetzt.

## Claims

1. A process for the production of L-proline from an L-pyroglutamic acid ester, characterised in that:

(*a*) an L-pyroglutamic acid ester corresponding to the general formula:

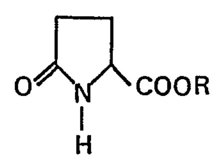

(I)

wherein R represents a methyl or ethyl radical, is reacted in an inert solvent, with at least 2 mol of phosgene per mol of the compound corresponding to the general formula (I) which is used, and the solvent and excess phosgene is subsequently separated by distillation;

(*b*) the residue resulting from reaction stage (*a*) is heated to a temperature of from 60 to 100°C for from 3 to 10 h in an inert solvent, with at least 1 mol of an acid acceptor per mol of the compound corresponding to the general formula (I) which was originally used;

(*c*) the crude reaction mixture resulting from reaction stage (*b*), or a compound, which has been isolated from the reaction mixture by distillation and dissolved in an inert solvent, corresponding to the general formula:

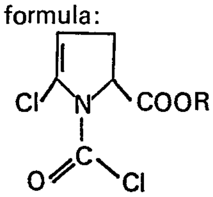

(III)

wherein R again represents a methyl or ethyl radical, is hydrogenated at a temperature of from 0 to 100°C and under a hydrogen pressure of from 1

to 300 bar, in the presence of a hydrogenation catalyst and at least a stoichiometric quantity of an acid acceptor, and the hydrogenation catalyst is subsequently separated by filtration;

(*d*) the crude reaction mixture resulting from reaction stage (*c*), or a compound, which has been isolated from the reaction mixture by distillation, corresponding to the general formula:

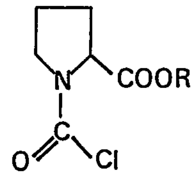

(IV)

wherein R again represents a methyl or ethyl radical, is hydrolysed with an aqueous mineral acid or a mixture of an aqueous mineral acid and formic or acetic acid, and

(*e*) the resulting L-proline is isolated in known manner from the hydrolysis mixture resulting from reaction stage (*d*).

2. A process according to Claim 1, characterised in that reaction stage (*a*) is carried out at a temperature of from −30 to +5°C and phosgene is added in condensed form.

3. A process according to Claim 1, characterised in that reaction stage (*a*) is carried out at a temperature of from 5 to 50°C and phosgene is introduced in gaseous form.

4. A process according to one of Claims 1 to 3, characterised in that the phosgene is used in a quantity of from 2.0 to 12 mol per mol of the compound corresponding to the general formula (I) which is used.

5. A process according to one of Claims 1 to 4, characterised in that a tertiary amine is used as acid acceptor in reaction stage (*b*).

6. A process according to one of Claims 1 to 5, characterised in that a metal from the 8th subsidiary group of the Periodic System, or a compound of a metal of this type, is used as the hydrogenation catalyst in reaction stage (*c*).

7. A process according to one of Claims 1 to 6, characterised in that a tertiary amine is used as acid acceptor in reaction stage (*c*).

8. A process according to one of Claims 1 to 7, characterised in that aqueous hydrochloric acid or a mixture of aqueous hydrochloric acid and formic or acetic acid is used as hydrolysing agent in reaction stage (*d*).

## Revendications

1. Procédé de préparation de L-proline à partir d'un ester de l'acide L-pyroglutamique, caractérisé en ce que:

a) on fait réagir un ester L-pyroglutamique de formule générale:

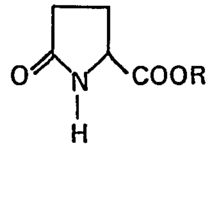

(I)

dans laquelle R signifie un groupe méthyle ou éthyle, dans un solvant inerte, avec au moins 2 mol de phosgène par mole de composé de formule générale (I) mise en œuvre, et, ensuite, on sépare par distillation le solvant et le phosgène en excès;

b) on fait chauffer le résidu obtenu dans l'étape réactionnelle a dans un solvant inerte, avec au moins 1 mol d'un accepteur d'acide par mole de composé de formule générale (I) mise en œuvre initialement, pendant 3 à 10 h, à une température comprise entre 60 et 100°C;

c) le mélange réactionnel brut obtenu dans l'étape réactionnelle b, ou le composé obtenu à partir de ce mélange par distillation et dissous dans un solvant inerte, répondant à la formule générale:

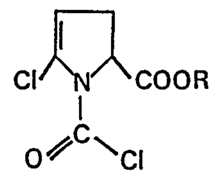

(III)

dans laquelle R signifie à nouveau un groupe méthyle ou éthyle, est hydrogéné en présence d'un catalyseur d'hydrogénation et d'une proportion au moins stœchiométrique d'un accepteur d'acide, à une température comprise entre 0 et 100°C et sous une pression d'hydrogène de 1 à 300 bar, puis le catalyseur d'hydrogénation est séparé par filtration;

d) le mélange réactionnel brut obtenu dans l'étape réactionnelle c, ou le composé obtenu à partir de ce mélange par distillation, répondant à la formule générale:

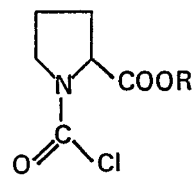

(IV)

dans laquelle R signifie à nouveau un groupe méthyle ou éthyle, est hydrolysé avec un acide minéral aqueux ou un mélange d'un acide minéral aqueux avec de l'acide formique ou acétique, et

e) on isole de manière connue en soi la L-proline du mélange d'hydrolyse obtenu dans l'étape réactionnelle d.

2. Procédé selon la revendication 1, caractérisé en ce que l'étape réactionnelle a est réalisée à une température comprise entre −30 et +5°C et que le phosgène est introduit sous forme condensée.

3. Procédé selon la revendication 1, caractérisé en ce que l'étape réactionnelle a est effectuée à une température comprise entre 5 et 50°C et que l'on introduit le phosgène sous forme de gaz.

4. Procédé selon l'une des revendications 1 à 3, caractérisé en ce que le phosgène est mis en œuvre dans une proportion comprise entre 2 et 12 mol par mole de composé de formule générale (I) mis en œuvre.

5. Procédé selon l'une des revendications 1 à 4, caractérisé en ce que dans l'étape réactionnelle b l'accepteur d'acide utilisé est une amine tertiaire.

6. Procédé selon l'une des revendications 1 à 5,

caractérisé en ce que dans l'étape réactionnelle c le catalyseur d'hydrogénation utilisé est un métal du sous-groupe VIII du système périodique, ou un composé d'un tel métal.

7. Procédé selon l'une des revendications 1 à 6, caractérisé en ce que dans l'étape réactionnelle c l'accepteur d'acide utilisé est une amine tertiaire.

8. Procédé selon l'une des revendications 1 à 7, caractérisé en ce que dans l'étape réactionnelle d l'agent d'hydrolyse utilisé est de l'acide chlorhydrique aqueux ou un mélange d'acide chlorhydrique aqueux et d'acide formique ou acétique.